**Europäisches Patentamt**

**European Patent Office**

**Office européen des brevets**

(19)

(11) Numéro de publication: **0 139 574**
**B1**

(12) # FASCICULE DE BREVET EUROPÉEN

(45) Date de publication du fascicule du brevet:
**08.06.88**

(51) Int. Cl.⁴: **A 61 B 8/12**, G 10 K 11/00

(21) Numéro de dépôt: **84401959.6**

(22) Date de dépôt: **01.10.84**

(54) Perfectionnement aux sondes d'échographie médicale endocavitaire.

(30) Priorité: **29.09.83 FR 8315522**

(43) Date de publication de la demande:
**02.05.85 Bulletin 85/18**

(45) Mention de la délivrance du brevet:
**08.06.88 Bulletin 88/23**

(84) Etats contractants désignés:
**AT BE CH DE FR GB IT LI LU NL SE**

(56) Documents cité:
**EP-A-0 028 825**
**EP-A-0 097 917**
**WO-A-83/00009**
**DE-A-3 219 118**
**FR-A-2 356 143**
**FR-A-2 424 733**
**FR-A-2 507 075**
**GB-A-2 073 418**

(73) Titulaire: **Fornage, Bruno Denis Lucien, Tramery,
F-51170 Fismes (FR)**
Titulaire: **Pourcelot, Léandre, 14, rue G. Guynemer
Saint- Avertin, F-37170 Chambray- les Tours (FR)**

(72) Inventeur: **Fornage, Bruno Denis Lucien, Tramery,
F-51170 Fismes (FR)**
Inventeur: **Pourcelot, Léandre, 14, rue G.
Guynemer Saint- Avertin, F-37170 Chambray- les
Tours (FR)**

(74) Mandataire: **Robert, Jean- Pierre, CABINET
BOETTCHER 23, rue la Boétie, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne une sonde d'échographie ultrasonore capable de fournir à l'opérateur, au cours d'une même manipulation, deux images tomographiques complémentaires d'une même région d'un corps entourant une cavité dans laquelle est introduite la sonde, ce corps creux pouvant être un liquide ou un solide inerte ou un corps vivant humain ou animal.

Dans le domaine médical le matériel existant, pour l'exploration endocavitaire par ultrasons, se divise en deux types de sondes.

Il s'agit tout d'abord de sondes endocavitaires à balayage mécanique. L'élément essentiel de ces sondes réside dans un capter pouvant tourner ou osciller autour d'un axe parallèle à ou confondu ave l'axe longitudinal de la sonde. Le champ exploré est défini par la surface engendrée, lors de la rotation du capteur, par son axe ultrasonore qui peut être soit orthogonal à l'axe de sa rotation soit incliné sur celui-ci. Ainsi cette surface est soit plane soit conique. Le champ exploré peut dans cette surface, être limité par l'amplitude de la rotation du capteur à un secteur angulaire déterminé ou au contraire s'étendre sur la totalité de cette surface.

On connaît également des sondes constituées par une pluralité de cristaux ou transducteurs élémentaires s'étendant sensiblement le long d'une génératrice de la sonde, c'est-à-dire parallèlement à on axe longitudinal. Cette pluralité de cristaux forme un capteur dit à balayage électroniuqe lnéaire ou sectoriel. La surface explorée est alors constituée par un plan, ou une portion regtangulaire de plan, passant par l'axe de la sonde. On peut ouvrir le champ exploratoire qui devient alors un secteur de plan plus ouvert que la portion regtangulaire susdite, soit par l'emploi d'une sonde légèrement incurvée portant à sa partie convexe ladite pluralité de cristaux, soit à partir d'un arrangement plan de transducteurs élémentaires, par déphasage des signaux électriques d'émission et de réception de chaque transducteur.

Il existe enfin des sondes comme décrites dans le document GB-A-2 073 418 qui comportent des cristaux circonférentiels adjacents à une extrémité du support et des cristaux frontaux parallèles à cette extrémité, ces derniers explorant la partie de cavité immédiatement devant la sonde.

On notera que dans ces types de sondes, les capteurs sont disposés à la partie terminale d'un support relativement rigide, le plus souvent cylindrique, destiné à assurer la mise en place et le déplacement des capteurs à l'intérieur de la cavité, notamment vaginale ou rectale à explorer.

Chaque sonde ne fournit qu'un seul type d'image soit contenant l'axe de la sonde, soit sécant avec cet axe. Or l'utilisation de l'échographie dans ce diagnostic médical requiert fondamentalement une représentation tomographique exhaustive des organes à l'aide de plans de coupe différents. Le minimum requis est de deux plans de coupe orthogonaux. La satisfaction de cette exigence implique, avec les dispositifs connus, le recours à l'introduction successive de deux sondes différentes pour obtenir, successivement dans le temps, les deux images tomographiques complémentaires.

Les inconvénients de l'utilisation de deux sondes successives résident d'une part dans l'inconfort pour le patient et d'autre part dans la nécessité de choisir et conserver des informations recueillies par la première sonde pour pouvoir effectuer une comparaison avec celles fournies par la seconde sonde.

La présente invention se propose de pallier ces inconvénients par le moyen d'une sonde unique. Cette sonde offre, outre les avantages de chacun des deux types rappelés ci-dessus, la possibilité d'une meilleure approche des organes dans l'espace et un repérage topographique optimal des lésions, contribuant ainsi au choix de la meilleure stratégie thérapeutique.

A cet effet, l'invention a pour objet une sonde d'échographie ultrasonore d'examen par voie endocavitaire, pour délivrer au moins deux images tomographiques complémentaires cette sonde comporte:
- un support sensiblement longiligne et cylindrique,
- une pluralité de transducteurs élémentaires formant un premier capteur à balayage électronique d'un premier plan d'observation,
    - un second capteur,
    - des moyens de connection électriques, électroniques et mécaniques s'étendant à l'intérieur dudit support, depuis lesdits capteurs jusqu'à d'une part un générateur de signaux d'excitation et, d'autre part, un appareil de visualisation des signaux recueillis,

dans laquelle la pluralité de transducteurs dudit premier capteur est disposée sensiblement le long d'une génératrice dudit support pour explorer une surface plane passant sensiblement par l'axe longitudinal dudit support tandis que ledit second capteur engendre un faisceau ultrasonore contenu dans une surface d'exploration sécante de ladite surface plane d'exploiration.

Dans un premier mode de réalisation, le premier capteur à balayage électronique est constitué par un arrangement plan de transducteurs élémentaires commandés par un dispositif électronique destiné à déphaser les signaux électroniques d'émission et de réception pour réaliser un balayage électronique sectoriel.

Dans une variante de ce mode de réalisation, ledit capteur à balayage électronique est constitué par un arrangement courbe de transducteurs élémentaires commandés par un dispositif électronique destiné à réaliser un balayage le long du support aboutissant à une déflexion angulaire du faisceau ultrasonore.

Par ailleurs, dans un mode de réalisation du second capteur, ce dernier est à balayage mécanique et constitué par un transducteur tournant dont l'axe ultrasonore est sensiblement perpendiculaire à son axe de rotation.

Dans une variante le capteur à balayage mécanique est constitué par un transducteur fixe, d'axe ultrasonore longitudinal, associé à un miroir acoustique tournant autour de l'axe longitudinal du support, d'inclinaison réglable surcet axe et attelé audit organe de transmission.

Un autre mode de réalisation du second capteur réside dans un arrangement plan de transducteurs élémentaires le long d'une direction sensiblement perpendiculaire à celle de l'arrangement du premier capteur et commandés électroniquement de manière à obtenir un balayage sectoriel par déphasage des signaux électriques d'émission et de réception.

On notera que ce second capteur peut être coplanaire au premier ou dans un plan formant un angle avec le plan du premier capteur.

Une variante de cet autre mode de réalisation consiste en un second capteur formé d'un arrangement courbe de transducteurs élémentaires le long d'une direction perpendiculaire à celle du premier capteur, commandés électroniquement pour réaliser un balayage transversal au support. On obtient alors une déflexion angulaire du faisceau ultrasonore.

L'invention sera mieux comprise au cours de la description donnée ci-après à titre d'exemple purement indicatif et non limitatif qui permettra d'en dégager les avantages et les caractéristiques secondaires.

Il sera fait référence aux dessins annexés dans lesquels:

- la figure 1 est un schéma général de la sonde selon l'invention,

- la figure 2 illustre par un schéma, un premier mode de réalisation de celle-ci, dans lequel le second capteur est à balayage mécanique,

- la figure 3 représente une variante de réalisation de la figure 2,

- la figure 4 est le schéma d'une variante de réalisation de la figure 3,

- la figure 5 est une représentation schématique d'un troisième mode de réalisation de l'invention,

- la figure 6 illustre par un schéma les différentes surfaces balayées par la sonde selon l'invention,

- les figures 7, 8 et 9 illustrent schématiquement des sondes dans lesquelles le second capteur est un arrangement de transducteurs élémentaires commandés électroniquement.

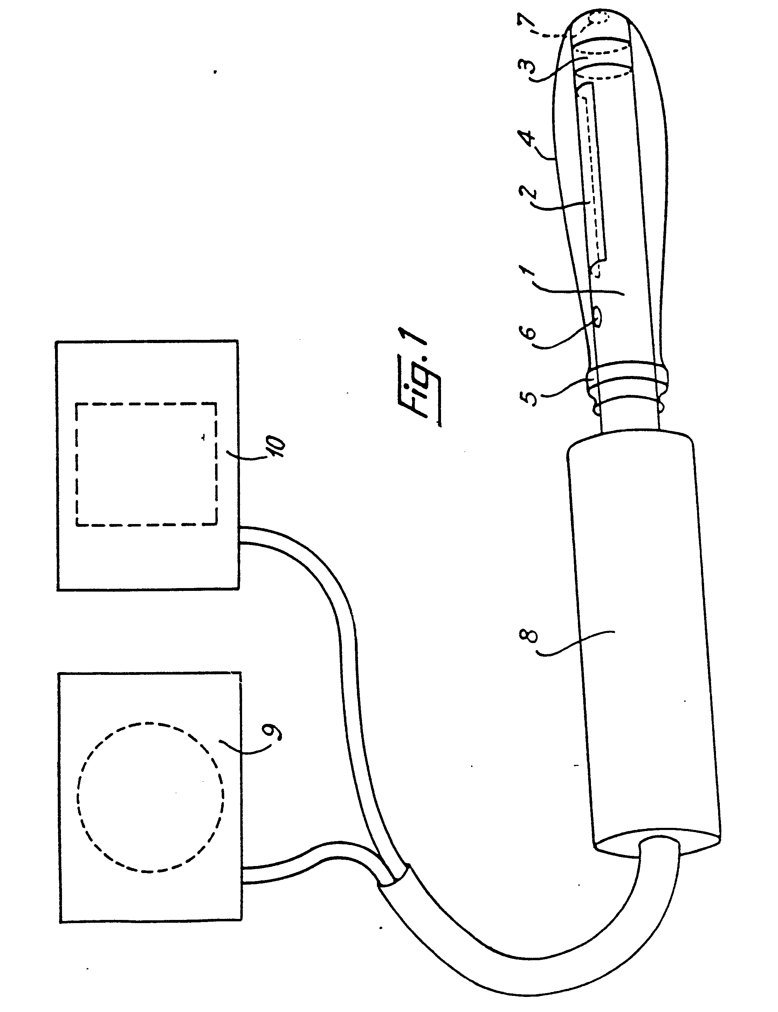

En se reportant tout d'abord à la figure 1, on voit une sonde constituée par un support 1 sensiblement cylindrique. Ce support, relativement rigide, est équipé d'une série de cristaux élémentaires 2 le long d'une de ses génératrices. Ces cristaux constituent un capteur à balayage électronique, connu en lui-même, qui permet d'explorer un demi-plan passant sensiblement par l'axe du support 1. Ce capteur s'étend sur la plus grande partie du support 1 tout en étant plutôt décalé vers sa zone distale.

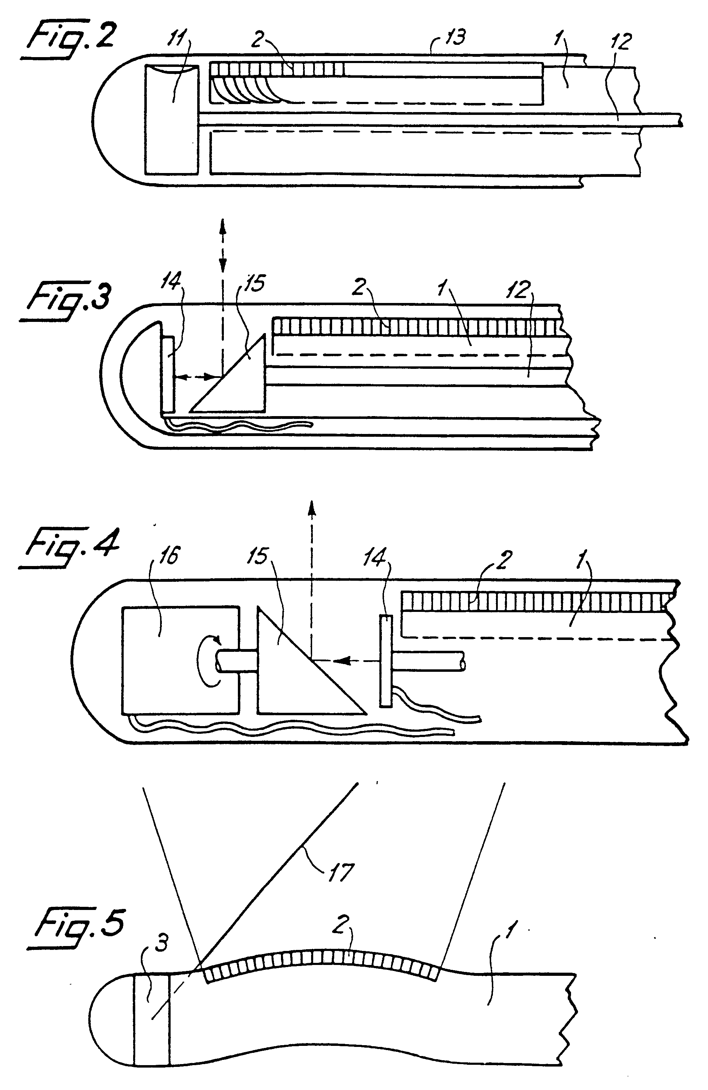

Au-delà de ce capteur, au voisinage de l'extrémité libre du support, on a représenté en 3 la zone dans laquelle est placé un second capteur ultrasonore capable d'explorer un plan sensiblement perpendiculaire à l'axe longitudinal du support par le moyen d'un balayage mécanique c'est-à-dire par rotation de l'axe ultrasonore du capteur perpendiculaire à l'axe longitudinal du support. Ce capteur de la zone 3 sera illustré plus en détail en regard des figures 2 à 4. L'ensemble du support 1, au capteur 2 et de la zone 3 est encapuchonné par un embout extérieur non représenté, qui protège les capteurs tout en constituant la forme extérieure du support, donc de la zone active de la sonde.

Le support comporte également une enveloppe 4 déformable, dans laquelle il est logé de manière étanche grâce à une collerette de fixation 5. Le volume intérieur de cette enveloppe peut être purgé ou alimenté en eau, au moyen de conduits disposés dans le support 1 et débouchant en 6 et 7 à sa surface.

Le support se prolonge d'une poignée 8 de manipulation permettant son introduction et son orientation dans la cavité à explorer, ainsi que la modification de cette orientation. Cette poignée comporte des boutons de commande du fonctionnement de la sonde ainsi que des index de rappel de l'orientation du capteur à balayage électronique linéaire.

Les connexions électriques et électroniques des capteurs 2 et de la zone 3 sont réalisés au moyen d'un câblage de connexion, s'étendant à l'intérieur du support 1, pour relier lesdits capteurs d'une part, à une source d'excitation des transducteurs qui forment les capteurs pour émettre par effet piézo-électrique les ultrasons et d'autre part, à un système de visualisation des signaux délivrés par les capteurs lorsqu'ils fonctionnent en récepteurs des échos ultrasoniques. Ce système de visualisation est représenté en 9 sur la figure 1 pour le capteur à balayage mécanique situé dans la zone 3 de la sonde et en 10 pour le capteur à balayage électronique linéaire. Il s'agira de préférence d'une visualisation vidéo.

Sur la figure 2 on retrouve certains des éléments de la figure 1 avec les mêmes références. On voit qu'ici le capteur à balayage mécanique est constitué par un transducteur 11 calé sur un arbre 12, susceptible d'être animé d'un mouvement de rotation au moyen d'un organe d'entraînement non représenté. Cet arbre 12 sera de préférence creux de manière que les conducteurs électriques puissent être logés à l'intérieur de celui-ci.

L'arbre 12 peut être soit rigide, dans le cas où une poignée de manipulation est disposée à proximité du support 1, le moteur étant alors logé dans cette poignée, soit souple et simplement guidé rigidement à l'intérieur du support 1 au niveau du capteur à balayage électronique linéaire 2. Cet arbre souple de transmission permet de disposer la sonde selon l'invention à l'extrémité d'un appareil du type endoscope, pour explorer des cavités éloignées de leur ouverture naturelle d'accès.

Par ailleurs on pourra prévoir que le transducteur 11 est brochable de manière amovible à l'extrémité de l'abre 12, ce qui permettra son interchangeabilité et ainsi de choisir le transducteur le mieux adapté à l'examen considéré.

Sur cette figure 2 on a également représenté le capuchon 13 évoqué en regard de la figure 1. Ce capuchon est amovible.

Ce n'est pas sortir du cadre de l'invention que de placer le transducteur tournant 11 entre deux séries égales et alignées de cristaux, qui forment le capteur à balayage électronique linéaire. Cette disposition offre l'avantage d'avoir des surfaces d'exploration par chacun des capteurs, toujours sécantes. Dans ce cas il est nécessaire de prévoir une synchronisation de l'activation des deux capteurs de manière à éviter que les signaux émis ou réfléchis de l'un viennent en interférence avec ceux de l'autre.

Sur la figure 3 on a représenté une variante de réalisation du capteur à balayage mécanique. Celui-ci est dans cet exemple, constitué par un transducteur fixe 14 dont l'axe ultrasonore est parallèle à l'axe du support 1. Ce transducteur est associé à un miroir acoustique 15 monté à rotation sur l'arbre de transmission 12. Ce miroir étant à 45°, le champ exploré est perpendiculaire à l'axe du support 1. On peut prévoir l'attelage du miroir 15 à l'arbre de transmission 12 réglable en inclinaison, de manière à transformer le champ exploré en une pluralité de surfaces coniques. De même que dans la figure précédente, le capteur à balayage mécanique constitué par le transducteur 14 et le miroir 15, peut être inséré au milieu du capteur à balayage électronique 2.

La figure 4, qui est une variante de réalisation de la figure 3, montre que le transducteur 14 fixe est situé entre le canteur à balayage électronique linéaire 2 et le miroir 15, contrairement à la disposition de la figure 3. Ainsi le miroir 15 peut être directement attelé en rotation à l'arbre de sortie d'un moteur 16 situé en partie distale de la sonde.

Sur la figure 5 on a représenté une sonde dont le support 1 est légèrement incurvé, avec un grand rayon de courbure, de manière que le capteur à balayage électronique linéaire 2 couvre, non plus une portion rectangulaire de plan, mais un secteur de plan. Cette disposition permet d'augmenter le champ exploratoire couvert par ce capteur 2.

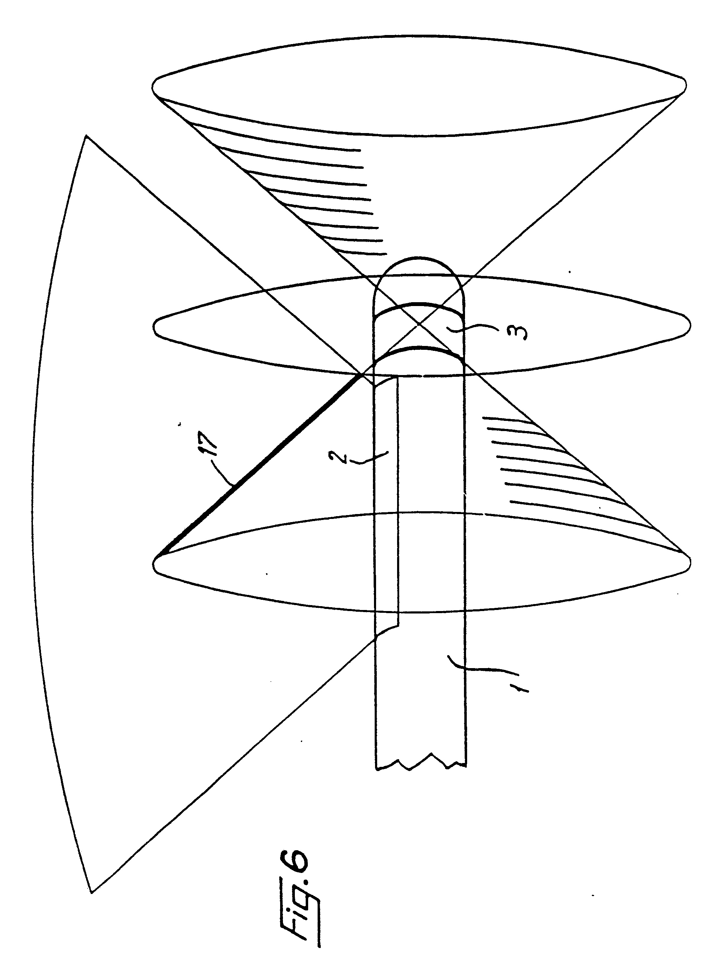

Sur cette figure 5 comme sur la figure 6 on a représenté schématiquement les surfaces explorées respectivement par le capteur à balayage électronique linéaire 2 et le capteur à balayage mécanique situé dans la zone 3. On voit sur ces figures, que lorsque le capteur à balayage mécanique engendre une surface d'exploration conique, dirigée vers l'arrière de la sonde, cette surface vient en intersection avec la surface explorée par le capteur à balayage électronique linéaire. La trace de cette intersection dans le plan couvert par le capteur 2 est représentée par la ligne 17. Cette intersection peut, par manipulation de la sonde, être placée précisément dans la zone à examiner dont on aura une image visualisée selon deux surfaces sensiblement orthogonales par les deux systèmes de visualisation 9 et 10. Les images ainsi recueillies permettront une évaluation beaucoup plus précise de l'étendue de la tumeur ou de la lésion examinée.

Le dispositif décrit ci-dessus en regard des figures 1 à 6 fait appel à une combinaison de deux capteurs à balayage mécanique d'une part et électronique d'autre part. Dans certaines applications cependant, il peut être avantageusement constitué par la combinaison de deux capteurs à balayage électronique.

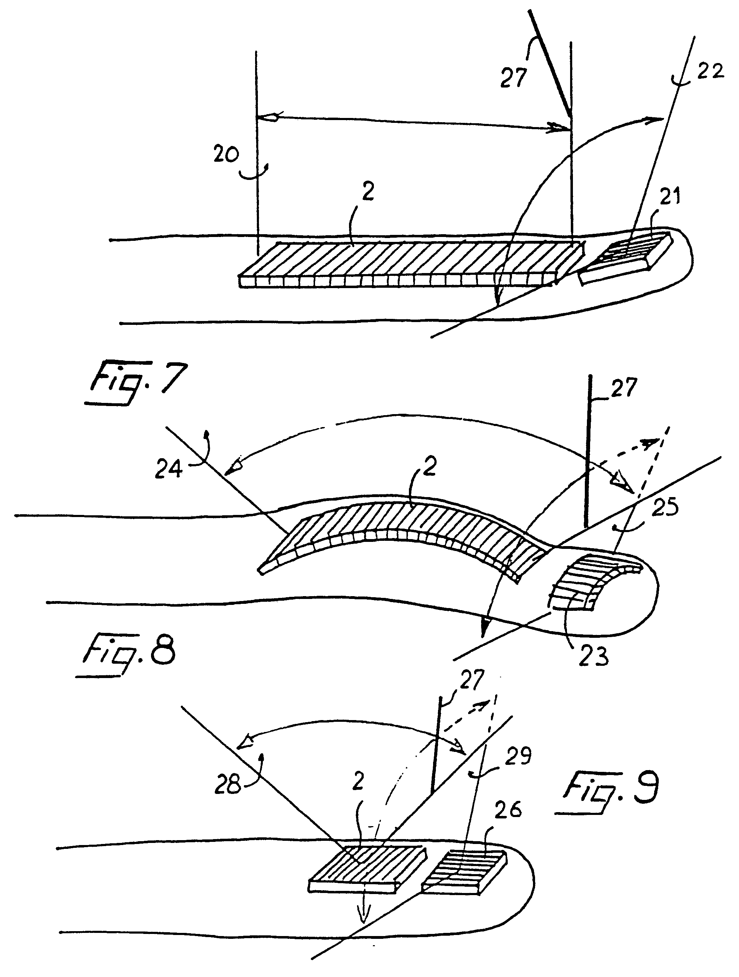

Les figures 7, 8 et 9 illustrent des dispositifs possédant deux ensembles de transducteurs disposés de manière à fournir deux plans de balayage perpendiculaires.

Ainsi sur la figure 7 on retrouve une série 2 (ou barrette) de transducteurs élémentaires qui permet au moyen d'une commutation électronique en commande de procéder au balayage du plan d'exploration 20. Une seconde série 21 de transducteurs est disposée à l'extrémité de la sonde dans un plan légèrement relevé par rapport au plan contenant la barrette 2. Au moyen d'une commande par dephasage électronique on peut balayer un secteur de plan 22 dont l'intersection avec le plan 20 est marquée par la ligne 27. Cette intersection étant placée dans la zone à explorer, on peut obtenir de cette zone une imagerie avantageuse en deux plans comme indiqué précédemment.

La figure 8 montre que l'on peut associer à une barrette courbe longitudinale, semblable à celle de la figure 5, une barrette courbe transversale 23. Les deux barrettes balayent respectivement les deux secteurs de plan 24 et 25 qui se coupent selon la ligne 27. Dans ce cas de figure il suffit de commander le balayage par commutation électronique pour néanmoins obtenir un balayage angulaire.

A la figure 9 enfin on a représenté deux barrettes planes 2 et 26 sensiblement perpendiculaires. Grâce à un déphasage électronique introduit dans leur commande, on réalise un balayage sectoriel dans les deux plans 28 et 29 se coupant le long de la ligne 27.

On notera que comme en regard des figures 2 à 6, le second capteur peut être logé à l'extrémité distale de la sonde ou logé entre deux portions de barrette longitudinale.

Il est possible grâce à l'invention d'obtenir une imagerie selon deux directions des zones examinées et ce soit alternativement soit simultanément.

En outre dans les diverses combinaisons décrites, il est possible d'étudier la variation de fréquence par effet Doppler des ondes ultrasonores réfléchies afin d'effectuer des mesures de vitesse d'écoulement des liquides (sang, urines...). On peut accéder à la mesure de débit, si l'image permet de déterminer le diamètre des conduits (artère, urètre...) dans lesquels circulent les fluides.

L'invention trouve une application non seulement dans le domaine médical mais également dans le domaine industriel pour l'exploration de solides ou liquides dans lesquels on peut introduire la sonde.

**Revendications**

1. Sonde d'échographie ultrasonore d'examen par voie endocavitaire pour délivrer deux images échographiques ultrasonores de deux plans d'exploration différents d'une même région du corps entourant une cavité dans laquelle est introduite ladite sonde, comprenant
- un support sensiblement longiligne et cylindrique,
- une pluralité de transducteurs élémentaires formant un premier capteur (2) à balayage électronique d'un premier plan d'observation,
- un second capteur (3, 11, 14, 15, 21, 25, 26),
- des moyens de connection électriques, électroniques et mécaniques s'étendant à l'intérieure dudit support (1), depuis lesdits capteurs jusqu'à d'une part un générateur de signaux d'excitation et, d'autre part, à un appareil (9), (10) de visualisation des signaux recueillis, caractérisée en ce que la pluralité de transducteurs dudit premier capteur (2) est disposée sensiblement le long d'une génératrice dudit support (1) pour explorer une surface plane passant sensiblement par l'axe longitudinal dudit support et en ce que ledit second capteur (3, 11, 14, 15, 21, 25, 26) engendre un faisceau ultrasonore contenu dans une surface d'exploration sécante de ladite surface plane d'exploration.

2. Sonde selon la revendication 1 caractérisée en ce que le premier capteur (2) susdit est constitué par un arrangement plan de transducteurs élémentaires commandés électroniquement de manière à déphaser les signaux électriques d'émission et de réception pour réaliser un balayage électronique sectoriel (28).

3. Sonde selon la revendication 1, caractérisée en ce que le premier capteur (2) est constitué par un arrangement courbe de transducteurs élémentaires dont la commande par commutation électronique permet de réaliser un balayage (24) le long du support aboutissant à une déflexion angulaire du faisceau ultrasonore.

4. Sonde selon l'une quelconque des revendications précédentes, caractérisée en ce que le second capteur susdit est constitué par un arrangement plan de transducteurs élémentaires (21, 26) commandés électroniquement de manière à déphaser les signaux électriques d'émission et de réception pour réaliser un balayage électronique sectoriel (22, 28).

5. Sonde selon l'une quelconque des revendications 1 à 4, caractrisée en ce que le second capteur susdit est constitué par un agencement courbe (26) de transducteurs élémentaires dont la commande par commutation électronique permet de réaliser un balayage (25) le long du support aboutissant à une déflexion angulaire du faisceau ultrasonore.

6. Sonde selon la revendication 1, caractérisée en ce que le second capteur est à balayage mécanique et est constitué par un transducteur (11) tournant, dont l'axe ultrasonore est sensiblement perpendiculaire à son axe de rotation.

7. Sonde selon la revendication 1, caractérisée en ce que le capteur est à balayage mécanique susdit et est constitué par un transducteur fixe (14) d'axe ultrasonore longitudinal, associé à un miroir acoustique (15) tournant autour de l'axe longitudinal du support (1), et d'inclinaison réglable sur ledit axe.

8. Sonde selon la revendication 7, caractérisée en ce que ledit miroir (15) est disposé entre le capteur à balayage électronique linéaire (2) et le transducteur (14).

9. Sonde selon la revendication 7, caractérisée en ce que le miroir (15) susdit est situé au-delà dudit transducteur (14) par rapport audit capteur à balayage électronique (2) et en ce que l'organe de transmission et l'organe d'entraînement (16) du miroir (15) sont situés au-delà de ce dernier.

10. Sonde selon l'une quelconque des revendications 1 à 9, caractérisée en ce que le second capteur est disposé en position distale (3) de la sonde par rapport au capteur à balayage électronique linéaire (2).

11. Sonde selon l'une quelconque des revendications 1 à 9, caractérisée en ce que le second capteur est disposé entre deux parties alignées du capteur à balayage électronique linéaire (2).

12. Sonde selon l'une quelconque des revendications 6 à 8, 10 et 11 caractérisée en ce que le capteur à balayage mécanique (11, 14, 15) est attelé à un organe de transmission de mouvement constitué par un arbre rigide (12) et en ce que l'organe d'entraînement est situé dans une poignée (8) de manipulation de la sonde solidaire du support susdit et pourvu des commandes dudit organe d'entraînement.

13. Sonde selon 1 une quelconque des revendications 6 à 8, 10 et 11, caractérisée en ce que le capteur à balayage mécanique est attelé à un arbre de transmission (12) souple et est guidé rigidement dans le support (1) au voisinage du capteur à balayage électronique linéaire (2).

14. Sonde selon la revendication 10, caractérisée en ce que le capteur à balayage mécanique (11) est connecte de manière amovible à l'organe de transmission (12).

15. Sonde selon l'une quelconque des revendications précédentes, caractérisée en ce qu'au moins le capteur à balayage mécanique est recouvert d'un capuchon (13) rigide et amovible conférant à la sonde sa forme extérieure.

16. Sode selon l'une quelconque des revendications précédentes, caractérisée en ce que le support et les capteurs sont contenus dans une enveloppe (4) souple et déformable, dans laquelle débouchent des canalisations (6) et (7) de remplissage et de purge de son volume intérieur ménagées dans ledit support.

**Patentansprüche**

1. Ultraschall-Echographiesonde zur Untersuchung eines inneren Hohlraumes durch Erzeugung zweier Ultraschall-Echographiebilder in zwei verschiedenen untersuchungsebenen ein und derselben, den Hohlraum umgebenden Körpergegend, in welchen die Sonde eingeführt ist, mit
- einem im wesentlichen langgestreckten und zylindrischen Träger,
- einer Mehrzahl elementarer Wandler, die einen ersten Aufnehmer (2) zur elektronischen Abtastung einer ersten Beobachtungsebene bilden,
- einem zweiten Aufnehmer (3, 11, 14, 15, 21, 25, 26) und
- elektrischen, elektronischen und mechanischen Verbindungsmitteln, die sich im Innern des Trägers (1) von den Aufnehmern einerseits zu einem Erregersignalgenerator und andererseits zu einem Apparat (9, 10) zur Sichtbarmachung der empfangenen Signale erstrecken,
dadurch gekennzeichnet,
daß die Mehrzahl der Wandler des ersten Aufnehmers (2) im wesentlichen entlang einer Erzeugenden des Trägers (1) angeordnet ist, um eine ebene Fläche zu untersuchen, die im wesentlich durch die Längsachse des Trägers verläuft, und
daß der zweite Aufnehmer (3, 11, 14, 15, 21, 25, 26) ein Ultraschallbündel erzeugt, das in einer die ebene Untersuchungsfläche schneidenden Untersuchungsfläche enthalten ist.
2. Sonde nach Anspruch 1, dadurch gekennzeichnet, daß der erste Aufnehmer (2) von einer ebenen Anordnung elementarer Wandler gebildet ist, die ihrerseits elektronisch derart gesteuert sind, daß sie die elektrischen Sende- und Empfangssignale phasenverschieben, um eine sektorielle elektronische Abtastung (28) zu realisieren.
3. Sonde nach Anspruch 1, dadurch gekennzeichnet, daß der erste Aufnehmer (2) von einer gekrümmten Anordnung elementarer Wandler gebildet ist, deren Steuerung durch elektronische Kommutation es gestattet, eine Abtastung (24) entlang dem Träger zu realisieren, die an eine Winkelablenkung des Ultraschallbündels angrenzt.
4. Sonde nach einem der voranstehenden Ansprüche, dadurch gekennzeichnet, daß der zweite Aufnehmer von einer ebenen Anordnung elementarer Wandler (21, 26) gebildet ist, die elektronisch derart gesteuert sind, daß sie die elektrischen Sende- und Empfangssignale phasenverschieben, um eine sektorielle elektronische Abtastung (22, 28) zu realisieren.
5. Sonde nach einem der Ansprüche 1 bis 4, dadurch gekennzeichnet, daß der zweite Aufnehmer von einer gekrümmten Anordnung (26) elementarer Wandler gebildet ist, deren Steuerung durch elektronische Kommutation es gestattet, eine Abtastung (25) entlang dem Träger zu realisieren, die an eine Winkelablenkung des Ultraschallbündels angrenzt.
6. Sonde nach Anspruch 1, dadurch gekennzeichnet, daß der zweite Aufnehmer eine mechanische Abtastung aufweist und von einem sich drehenden Wandler (11) gebildet ist, dessen Ultraschallachse im wesentlichen senkrecht zu seiner Drehachse verläuft.
7. Sonde nach Anspruch 1, dadurch gekennzeichnet, daß der Aufnehmer eine mechanische Abtastung aufweist und von einem ortsfesten Wandler (14) mit längsgerichteter ultraschallachse gebildet ist, dem ein akkustischer Spiegel (15) zugeordnet ist, der sich seinerseits um die Längsachse des Trägers (1) dreht und eine regulierbare Neigung auf dieser Achse besitzt.
8. Sonde nach Anspruch 7, dadurch gekennzeichnet, daß der Spiegel (15) zwischen dem Aufnehmer mit linearer elektronischer Abtastung (2) und dem Wandler (14) angeordnet ist.
9. Sonde nach Anspruch 7, dadurch gekennzeichnet, daß der Spiegel (15) jenseits des Wandlers (14) mit Bezug auf den Aufnehmer (2) mit elektronischer Abtastung liegt, und daß das Übertragungsorgan und das Antriebsorgan (16) des Spiegels (15) jenseits des letzteren liegen.
10. Sonde nach einem der Ansprüche 1 bis 9, dadurch gekennzeichnet, daß der zweite Aufnehmer in distaler Position (3) der Sonde mit Bezug auf den Aufnehmer (2) mit linearer elektronischer Abtastung angeordnet ist.
11. Sonde nach einem der Ansprüche 1 bis 9, dadurch gekennzeichnet, daß der zweite Aufnehmer zwischen zwei ausgefluchteten Teilen des Aufnehmers (2) mit linearer elektronischer Abtastung angeordnet ist.
12. Sonde nach einem der Ansprüche 6 bis 8, 10 und 11, dadurch gekennzeichnet, daß der Aufnehmer (11, 14, 15) mit mechanischer Abtastung an ein Übertragungs-Bewegungsorgan angekuppelt ist, das von einer starren Achse (12) gebildet ist, und daß das Antriebsorgan in einem Handgriff (8) zur Manipulation der Sonde liegt, der mit dem Träger fest verbunden und mit Steuerungen des Antriebsorganes versehen ist.
13. Sonde nach einem der Ansprüche 6 bis 8, 10 und 11, dadurch gekennzeichnet, daß der Aufnehmer mit mechanischer Abtastung an eine biegsame Übertragungswelle (12) angekuppelt und starr im Träger (1) in der Nähe des Aufnehmers (2) mit linearer elektronischer Abtastung geführt ist.
14. Sonde nach Anspruch 10, dadurch gekennzeichnet, daß der Aufnehmer (11) mit mechanischer Abtastung unbeweglich mit dem Übertragungsorgan (12) verbunden ist.

15. Sonde nach einem der voranstehenden Ansprüche, dadurch gekennzeichnet, daß wenigstens der Aufnehmer mit mechanischer Abtastung mit einer starren und unbeweglichen Kappe (13) abgedeckt ist, welche der Sonde ihre äußere Form verleiht.

16. Sonde nach einem der voranstehenden Ansprüche, dadurch gekennzeichnet, daß der Träger und die Aufnehmer in einer nachgiebigen und deformierbaren Hülle (4) enthalten sind, in welche Leitungen (6, 7) zur Füllung und Entleerung ihres im Träger ausgesparten Innenraumes ausmünden.

## Claims

1. Endocavitary ultrasound probe for delivering two ultrasonic views of two different scanning planes of a single region of the body surrounding a cavity in which said probe is inserted, including
- a substantially longitudinal and cylindrical support,
- a plurality of elementary transducers forming a first electronic scanning transducer (2) of a first plane of observation,
- a second transducer (3, 11, 14, 15, 21, 25, 26),
-electrical, electronical and mechanical connecting means extending inside said support from said transducers to, on one way, a source of excitation signals and on the other way, a visualization unit (5, 10) of the back signals, characterized in that:
- the plurality of transducers of said first scanning transducèr (2) is located substantially along a generative line of said support (1), for scanning a plane surface including substantially the longitudinal axis of said support and in that
- said second transducer (3, 11, 14, 15, 21, 25, 26) gerenates an ultrasonic beams included in a scanning surface which intersects said plane surface.

2. Probe according to claim 1, characterized in that, the first scanning transducer (2) is made up of an plane arrangement of elementary transducers electronically controlled so that emitted and received signals are phase-shifted in order to achieve a sector-plane scanning (28).

3. Probe according to claim 1, characterized in that said first scanning transducer (2) is made up of an curved arrangement of elementary transducers which are controlled by electronic commutation for achieving a scanning (24) along the support which results in angular def lection of the ultrasonic beam.

4. Probe according to anyone of the preceding claims characterized in that said second transducer is made up of a plane arrangement of elementary transducers (21, 26) electronically controlled for phase shifting emitted signals and received signals in order to achieve a plane sector scanning (22, 28).

5. Probe according to anyone of claims 1 to 4, characterized in that said second transducer is made up of an curved arrangement (26) of elementary transducers which are controlled by electronic commutation for acheiving a scanning (25) along the support which results in an angular deflection of the ultrasonic beam.

6. Probe according to claim 1, characterized in that said second transducer is a mechanical scanning transducer and comprises a rotable transducer (11), the ultrasonic axis of which being substantially perpendicular to its rotational axis.

7. Probe according to claim 1, characterized in that said transducer is a mechanical scanning transducer and comprises a stationary transducer (14) of which the ultrasonic axis is longitudinal, cooperating with an acoustic miror (15) which is rotated about the longitudinal axis of the support (1), and variably tilted with respect to said axis.

8. Probe according to the claim 7, characterized in that said miror (15) is located between the linear electronic scanning transducer (2) and the transducer (14).

9. Probe according to the claim 7, characterized in that said miror (15) is located beyond said transducer (14) with respect to said electronic scanning transducer (2) and in that transmission means and driving means (16) of the miror (15) are located beyond this latter.

10. Probe according to any one of claims 1 to 9 characterized in that the second transducer is located at the distal end (3) of the probe with respect to the linear electronic scanning transducer (2).

11. Probe according to anyone of the claims 1 to 9, characterized in that said second transducer is located between two alined parts of the linear electronic scanning transducer (2).

12. Probe according to anyone of the claims 6 to 8, 10 and 11, characterized in that the mechanical scanning transducer (11, 14, 15) is keyed on transmission means of movement which is a rigid shaft (12) and in that driving means are housed in a handle (8) of the probe solid with said support and provided with control means of said driving means.

13. Probe according to anyone of claims 6 to 8, 10 and 11, characterized in that the mechanical scanning transducer is keyed on a flexible transmission shaft (12) and is rigidly guided in said support (1) in the vicinity of the linear electronic scanning transducer (2).

14. Probe according to claim 10, characterized in that the mechanical scanning transducer (11) is detachably connected to transmission means (12).

15. Probe according to anyone of the preceding claims, characterized in that at least the mechanical scanning transducer is removably housed in a rigid cap (13) giving the probe its external shape.

16. Probe according to anyone of the preceding claims, characterized in that at least the mechanical scanning transducer is removabley housed in a rigid cap (13) giving the probe its external shape.

16. Probe according to anyone of the preceding claims, characterized in that the support and the transducer are housed in a soft deformable envelope (4) into which ducts (6) and (7) managed in the support open for filling and draining the internal volume of the envelope.

_Fig.1_

0 139 574

Fig.2

Fig.3

Fig.4

Fig.5

0 139 574

Fig.6

_Fig.7_

_Fig.8_

_Fig.9_